# EUROPÄISCHE PATENTANMELDUNG

(11) **EP 0 697 202 A2**
(43) Veröffentlichungstag der Anmeldung: **21.02.1996**
(21) Anmeldenummer: 95890135.7
(22) Anmeldetag: 12.07.1995
(51) Int. Cl.: A61F 13/20

(54) **Verwendung eines getränkten Tampons**

(30) Priorität: 20.07.1994 AT 1437/94
(71) Anmelder: Schmid, Christine, A-2130 Mistelbach (AT)
(72) Erfinder: Schmid, Christine, A-2130 Mistelbach (AT)
(74) Vertreter: Patentanwälte BARGER, PISO & PARTNER

(57) **Zusammenfassung**

Die Erfindung betrifft die Verwendung eines mit Paraffinum liquidum (Vaselinöl) oder mit einem anderen hydrophoben, pharmakologisch unbedenklichem und bei Körpertemperatur flüssigen oder zumindest dickflüssigen (viskosen) Medium (Imprägniersubstanz ) getränkten Tampons zur Verhinderung bzw. Verminderung des Eindringens von Wassser in den Scheidengang beim Baden oder Schwimmen.

Ein erfindungsgemäßes Verfahren zum Tränken eines Tampons für die erfindungsgemäße Verwendung dadurch gekennzeichnet, daß der Tampon für etwa 2 Minuten in die Imprägniersubstanz eingelegt wird und anschließend etwa 2 Minuten lang abtropfen lassen wird.

## Beschreibung

Die Erfindung betrifft die Verwendung eines mit Paraffinum liquidum (früher Vaselinöl genannt) oder mit einem anderen hydrophoben, pharmakologisch unbedenklichem und bei Körpertemperatur flüssigen oder zumindest dickflüssigen (viskosen) Medium (Imprägniersubstanz) getränkten Tampons zur Verhinderung des Eindringens von Wasser in den Scheidengang beim Baden oder Schwimmen.

Das Tränken oder Imprägnieren von Tampons mit Paraffinum liquidum und anderen Stoffen zur Verbesserung der Gleitfähigkeit des Tampons und somit zum Erleichtern seines Einführens ist beispielsweise aus der US-PS 3 428 044, der US-PS 3 335 726, der US-PS 3 796 219, der US-PS 3 595 236 und der DE-OS 23 21 086 bekannt. In diesen Druckschriften wird noch die früher gebräuchliche Bezeichnung "Vaselin" bzw. "Vaselinöl" verwendet, ebenso in Römpps Chemie Lexikon, achte Auflage.

Darüberhinaus ist es aus der DE-PS 19 25 086 und der DE-OS 20 24 930 bekannt, Tampons unter anderem mit mineralischem Öl als Träger eines Medikamentes zu tränken, um das Medikament in genau vorgegebener Menge und Konzentration ins Innere des Scheidenganges einzubringen.

Schließlich ist es aus der DE-OS 1 541 275 bekannt, mit Kamillenabsud od.dergl. getränkte Scheidentampons, gegebenenfalls in Verbindung mit einem Gleitmittel (Glykol, Glyzerin), während des Badens oder Schwimmens zu verwenden. Ohne Gleitmittel ist das Einführen des sudgetränkten Tampons nicht unproblematisch, mit dem Gleitmittel ist die Vorbereitung des Tampons vor dem Einführen aufwendig und der vorbereitete Tampon schlecht lagerbar.

Gegenüber dem vorbekannten Stand der Technik ist es die Aufgabe der Erfindung, das Eindringen von Wasser in den Scheidengang beim Baden oder Schwimmen zu verhindern und darüberhinaus den Kontakt des Scheidenepithels mit eventuell doch eingedrungenem kontaminierten Wasser zu unterbinden oder möglichst vollständig zu verhindern, wobei sowohl die Vorbereitung des Tampons und die Lagerfähigkeit des vorbereiteten Tampons als auch das Einführen des Tampons erleichtert werden soll.

Erfindungsgemäß geschieht dies dadurch, daß ein Tampon in die Scheide eingeführt wird, der zuvor unter normalen Umgebungsbedingungen und im wesentlichen bei Raumtemperatur während einiger Minuten in Paraffinum liquidum (wie es in der Medizin in Verwendung steht), oder eine andere hydrophobe, pharmakologisch unbedenkliche und bei Körpertemperatur flüssige oder dickflüssige Substanz getaucht wird, sodaß er während dieser Zeit mit der Substanz getränkt wird.

Nach dem Einführen des so vorbereiteten Tampons in die Scheide verhindert einerseits der getränkte Tampon zumindest weitestgehend das Eindringen von Wasser in den Scheidenkanal und sorgt andererseits durch die Ausbildung eines Überzuges aus Paraffinum liquidum bzw. aus der Substanz auf dem Epithel des Scheidenganges dafür, daß etwa doch in den Scheidengang eingedrungenes Wasser nicht in direkten Kontakt mit dem Epithel gelangt.

Während bei der vorbekannten erstgenannten Verwendung als Gleitschutz möglichst wenig Paraffinum liquidum oder verwandte Stoffe verwendet werden sollten, um die Quellfähigkeit des Tampons nicht zu behindern und während bei der vorbekannten zweitgenannten Anwendung angestrebt wurde, ein Medikament möglichst innig und direkt in Kontakt mit der Epithels des Scheidenganges zu bringen, strebt die Erfindung ganz im Gegenteil einen weitestgehenden Abschluß und eine Abdeckung des Epithels des Scheidenganges gegenüber äußeren Einflüssen an. Bei der vorbekannten drittgenannten Anwendung wird zwar ebenfalls der erfindungsgemäß angestrebte Zweck angestrebt, doch geschieht dies mit aufwendigeren und dennoch nicht so wirksamen Methoden und Mitteln wie bei der Erfindung.

Als Beispiel für die erfindungsgemäße Vorbehandlung eines Tampons können folgende Werte als Richtschnur gelten:

Gewicht des trockenen Tampons: etwa 2,7 g; Gewicht nach 2-minütigem Einlegen in "Paraffinum liquidum" und 2-minütiger Abtropfzeit: etwa 7,8 g.

Als Imprägniersubstanz kann insbesonders auch eine der folgenden Substanzen oder eine Mischung davon und/oder mit Paraffinum liquidum verwendet werden: Glyzerin, pflanzliche Öle, tierische Öle, mineralische Öle und höhere aliphatische Alkohole.

## Patentansprüche

1. Verwendung eines mit Paraffinum liquidum (Vaselinöl) oder mit einem anderen hydrophoben, pharmakologisch unbedenklichem und bei Körpertemperatur flüssigen oder zumindest dickflüssigen (viskosen) Medium (Imprägniersubstanz) getränkten Tampons zur Verhinderung bzw. Verminderung des Eindringens von Wassser in den Scheidengang beim Baden oder Schwimmen.

2. Verfahren zum Tränken eines Tampons für die Verwendung nach Anspruch 1, dadurch gekennzeichnet, daß der Tampon für etwa 2 Minuten in die Imprägniersubstanz eingelegt wird und anschließend etwa 2 Minuten lang abtropfen lassen wird.

3. Verfahren nach Anspruch 2, dadurch gekennzeichnet, daß der Tampon nach dem Abtropfen etwa 5 g der Imprägniersubstanz aufgenommen hat.
